# EUROPEAN PATENT APPLICATION

(11) **EP 4 272 675 A1**
(43) Date of publication of application: **08.11.2023**
(21) Application number: 21913388.1
(22) Date of filing: 26.10.2021
(51) Int. Cl.: A61B 18/14, A61B 18/12, G16H 40/60, G16H 50/70

(54) **ABLATION PARAMETER CONFIGURATION METHOD, APPARATUS AND SYSTEM, AND COMPUTER-READABLE STORAGE MEDIUM**

(30) Priority: 31.12.2020 CN 202011639972
(71) Applicant: Hangzhou Broncus Medical Co., Ltd., Hangzhou, Zhejiang 310051 (CN)
(72) Inventor: CUI, Changjie, Hangzhou, Zhejiang 310051 (CN); XU, Hong, Hangzhou, Zhejiang 310051 (CN)
(74) Representative: Petraz, Gilberto Luigi
(86) International application number: PCT/CN2021/126360
(87) International publication number: WO 2022/142643

(57) **Abstract**

An ablation parameter configuration method, apparatus and system, and a computer-readable storage medium, wherein the method includes: creating an ablation task in response to a task creation instruction; acquiring a target file to which the task creation instruction points and extracting ablation parameters from the target file, wherein the target file includes an ablation scheme of an ablation object to which the task creation instruction points; associating the extracted ablation parameters with the ablation task, and recording an association relationship between the ablation parameters and the ablation task; and acquiring target ablation parameters of a target ablation task to which a task execution instruction points according to the recorded association relationship when the task execution instruction is triggered, and configuring the target ablation parameters into a radio frequency ablation system. The above ablation parameter configuration method, apparatus and system, and the computer-readable storage medium can realize automatic configuration of the RFA system, thereby simplifying the parameter configuration operation, saving the configuration time and improving the configuration efficiency.

## Description

### TECHNICAL FIELD

Embodiments of the present application relate to the technical field of communication, and particularly to an ablation parameter configuration method, apparatus and system, and a computer-readable storage medium.

### BACKGROUND

Radio frequency ablation (RFA) technology is a relatively common technology of minimally invasive tumor ablation. The principle of the RFA is applying a high-frequency alternating current with a frequency less than 30MHz (megahertz) to make ions in tumor tissues oscillate at a high speed, rub each other to convert radio frequency energy into heat energy, thereby causing coagulative necrosis of the tumor cells.

Before executing an ablation task, there is a need to configure parameters of the RFA system. Currently, parameter configuration is mainly done manually. However, due to the multiple parameters of the RFA system, the multiple stages involved from determining the ablation scheme to executing the ablation task, and each stage requiring an operation of inputting parameters, it is time consuming, cumbersome and prone to errors. Therefore, how to simplify parameter configuration operations, save configuration time, and improve configuration efficiency is a major problem that needs to be solved urgently in the industry.

### TECHNICAL PROBLEM

Embodiments of the present application provides an ablation parameter configuration method, apparatus and system, and a computer-readable storage medium, which can realize automatic parameter configuration of a radio frequency ablation system, simplify parameter configuration operations, save configuration time, and improve configuration efficiency.

### TECHNICAL SOLUTION

On one aspect, an embodiment of the present application provides an ablation parameter configuration method, which is applied to a computer terminal, including:
creating an ablation task in response to a task creation instruction;
acquiring a target file to which the task creation instruction points and extracting ablation parameters from the target file, wherein the target file includes an ablation scheme of an ablation object to which the task creation instruction points;
associating the extracted ablation parameters with the ablation task, and recording an association relationship between the ablation parameters and the ablation task;
acquiring target ablation parameters of a target ablation task to which a task execution instruction points according to the recorded association relationship when the task execution instruction is triggered, and configuring the target ablation parameters into a radio frequency ablation system.

On another aspect, an embodiment of the present application further provides an ablation parameter configuration device, including:
a creation module, configured for creating an ablation task in response to a task creation instruction;
an extraction module, configured for acquiring a target file to which the task creation instruction points and extracting ablation parameters from the target file, wherein the target file includes an ablation scheme of an ablation object to which the task creation instruction points;
a recording module, configured for associating the extracted ablation parameters with the ablation task and recording an association relationship between the ablation parameters and the ablation task; and
a configuration module, configured for acquiring target ablation parameters of a target ablation task to which a task execution instruction points according to the recorded association relationship when the task execution instruction is triggered and configuring the target ablation parameters into a radio frequency ablation system.

On another aspect, an embodiment of the present application further provides an electronic apparatus that includes a memory and a processor;
the memory storing executable program codes thereon;
the processor being electrically coupled to the memory, calling the executable program codes stored on the memory and executing the ablation parameter configuration method provided in the above embodiment.

On another aspect, an embodiment of the present application further provides an ablation parameter configuration system that includes a radio frequency ablation system, a parameter configuration device and a database server;
the radio frequency ablation system including a radio frequency ablation control device, a radio frequency ablation catheter, a neutral electrode and an injection pump;
the parameter configuration device being configured for executing steps of the ablation parameter configuration method provided in the above embodiment.

On another aspect, an embodiment of the present application further provides a radio frequency ablation system that includes a radio frequency ablation control device, a radio frequency ablation catheter, a neutral electrode and an injection pump;
the radio frequency ablation control device or the injection pump being configured for executing steps of the ablation parameter configuration method provided in the above embodiment.

On another aspect, an embodiment of the present application further provides a computer-readable storage medium on which a computer program is stored, wherein the computer program, when executed by a processor, implements the ablation parameter configuration method provided in the above embodiment.

### BENEFICIAL EFFECT

From the above embodiments of the present application, it can be seen that the present application associates the ablation parameters extracted from the ablation scheme of the target file with the created ablation task by means of utilizing the target file to which the task creation instruction points, and configures the target ablation parameters of the target ablation task to which the task execution instruction points into the radio frequency ablation system according to the recorded association relationship when the task execution instruction is triggered, thereby realizing automatic configuration of ablation parameters. Since the configuration parameters are automatically extracted from the target file which needs to be generated only once by the configuration device and configured into the radio frequency ablation system, on the basis of simplifying the parameter configuration operation, it can also avoid repetitive labor, save configuration time, and improve configuration efficiency.

### BRIEF DESCRIPTION OF THE DRAWINGS

In order to describe the technical solutions according to the embodiments of the present application or in the prior art more clearly, the drawings needed to be used in the embodiments or in the prior art will be described briefly below. Apparently, the drawings in the following description show some embodiments of the present application. Other drawings can be obtained by persons of ordinary skill in the art based on these drawings without creative efforts.
FIG. 1 is a diagram showing an application environment of an ablation parameter configuration method provided by an embodiment of the present application.
FIG. 2 is a flowchart of an ablation parameter configuration method provided by an embodiment of the present application.
FIG. 3 is a flowchart of an ablation parameter configuration method provided by another embodiment of the present application.
FIG. 4 is a schematic view of an ablation parameter configuration device provided by an embodiment of the present application.
FIG. 5 is a schematic view of a hardware structure of an electronic apparatus provided by an embodiment of the present application.
FIG. 6 is a schematic view of an ablation parameter configuration system provided by an embodiment of the present application.
FIG. 7 is a schematic view of a radio frequency ablation system provided by an embodiment of the present application.

### DESCRIPTION OF THE EMBODIMENTS

In order to make the objects, technical solutions and advantages of the embodiments of the present application clearer, the technical solutions according to the embodiments of the present application will be clearly and completely described with reference to drawings in the embodiments of the present application. Apparently, the embodiments described are merely some embodiments, but not all of the embodiments of the present application. All other embodiments obtained by ordinary persons skilled in the art based on the embodiments of the present application without creative efforts shall fall within the protection scope of the present application.

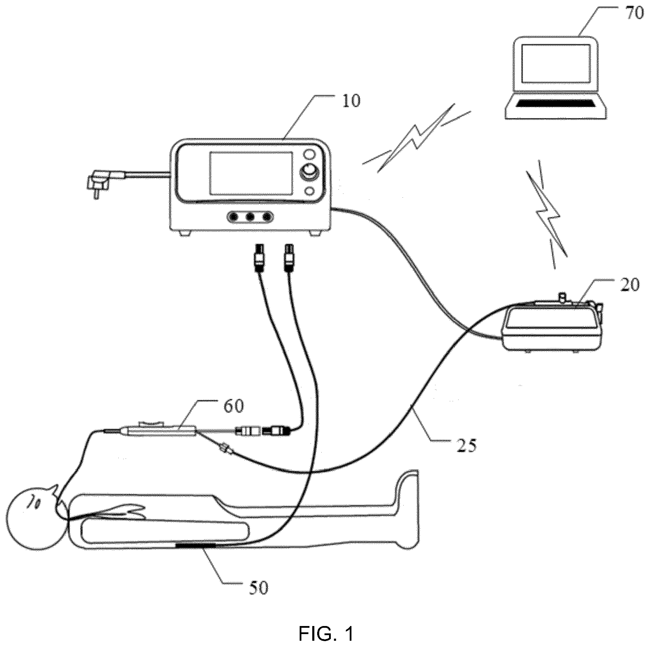

Referring to FIG. 1, which is a schematic diagram of an application environment of an ablation parameter configuration method provided by an embodiment of the present application. As shown in FIG. 1, a radio frequency ablation (RFA) system includes a RFA control device 10, an injection pump 20, a neutral electrode 50 and a RFA catheter 60. The ablation parameter configuration method may be implemented by a parameter configuration device 70 shown in FIG. 1. Alternatively, the ablation parameter configuration method may be implemented by the RFA control device 10 or the injection pump 20 shown in FIG. 1. The parameter configuration device 70 is a computer device that can perform data processing on or off the move, such as a server, a desktop computer, a notebook computer, a laptop computer, a tablet computer, a personal computer, a smart phone and etc.

Taking parameter configuration of the RFA system through the parameter configuration device 70 as an example, in practical applications, the parameter configuration device 70 establishes a data connection with at least one of the RFA control device 10 and the injection pump 20 through wireless or wired networks or data cables.

It can be understood that: when the parameter configuration device 70 establishes a connection with only one of the RFA control device 10 and the injection pump 20, parameter configuration control instructions and the parameters to be configured may be transmitted to another device connected to the connected device through the connected device, thereby achieving parameter configuration.

Before executing the ablation task, firstly, the RFA catheter 60 that is used to generate and output RF energy and an extension tube 25 of the injection pump 20 are inserted into the body of an ablation object (such as a lung cancer patient) and reach an ablation site; and then, the neutral electrode 50 is attached to a skin surface of the ablation object. Radio frequency current flows through the RFA catheter 60, tissues of the ablation object and the neutral electrode 50, forming a circuit.

The parameter configuration device 70 performs parameter configuration on at least one of the RFA control device 10, the injection pump 20, the neutral electrode 50 and the RFA catheter 60 of the RFA system by executing the ablation parameter configuration method provided in the following embodiments.

When an ablation task is triggered, the RFA control device 10 controls the RFA catheter 60 to output RF energy to the ablation site in a manner of unipolar discharging according to the ablation parameters configured by the parameter configuration device 70, so as to execute an ablation operation on the ablation site. Meanwhile, the injection pump 20 executes a perfusion operation on the ablation object through the extension tube 25 according to ablation parameters configured by the parameter configuration device 70, thereby perfusing physiological saline to the ablation site.

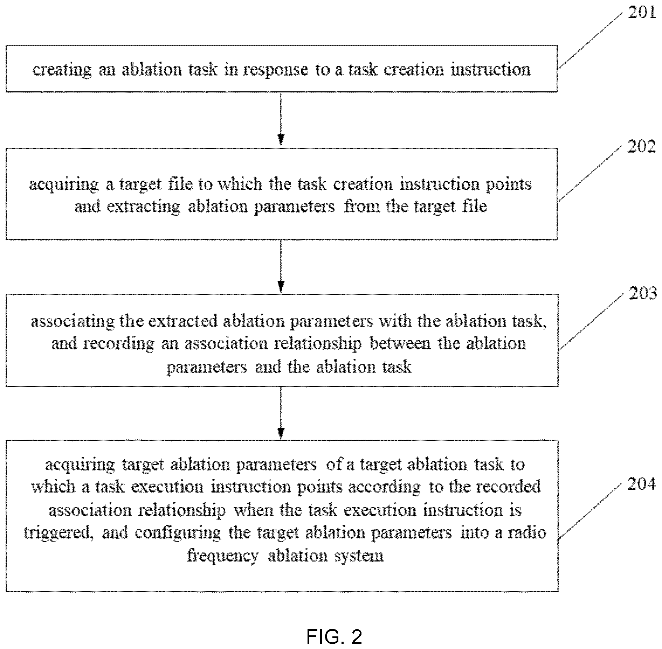

Referring to FIG. 2, which is a flowchart of an ablation parameter configuration method provided by an embodiment of the present application. The method may be implemented by a computer terminal, such as the parameter configuration device 70 or the RFA control device 10 or the injection pump 20 shown in FIG. 1, which is hereinafter referred to as a configuration device for ease of understanding. As shown in FIG. 2, the method includes:
Step S201: creating an ablation task in response to a task creation instruction;

Specifically, the task creation instruction may be sent to the configuration device from a server or other terminals. Alternatively, the task creation instruction may be triggered based on the user's operations on a task creating interaction interface provided by the configuration device.

The configuration device responds to the task creation instruction, creates the ablation task, and assigns identification information to the created ablation task. This identification information is used to uniquely identify the created ablation task, which may be consisted of but not limited to at least one of numbers, letters, Chinese characters, and special symbols. Furthermore, the identification information may include, but is not limited to at least one of identification information of the ablation object, creating time and characteristics of the ablation site.

Step S202, acquiring a target file to which the task creation instruction points and extracting ablation parameters from the target file;
Specifically, the target file may be stored on a remote server or client, as well as in the configuration device locally or other mobile storage devices. The target file includes an ablation scheme of an ablation object to which the task creation instruction points.

The ablation scheme may be generated based on data inputted in electronic sheets provided by users with pre-authorization permissions (such as doctors) on another electronic terminal. In a practical application, the other electronic terminal generates a pre-formatted target file containing the ablation scheme based on the user's input operation, and the target file is stored on a database server. A format of the target file may be but is not limited to any one of: word, txt, xml, CSV, DAT, DBF, MDB, ODB++.

Alternatively, the target file may be an image file of the user's handwritten prescription form. The configuration device may acquire the ablation scheme contained in the image file by utilizing file recognition technology.

Step S203: associating the extracted ablation parameters with the ablation task, and recording an association relationship between the ablation parameters and the ablation task;
Specifically, the recorded association relationship may be stored on the configuration device locally, or on the database server in the form of database files, or in the control device of the RFA system.

It can be understood that the database server stores the association relationship between at least one ablation task created by the configuration device and its respective ablation parameters. It may be understood that one task creation instruction may be used to create at least one ablation task.

In addition to storage, the database server further provides data query services based on the association relationship for the configuration device and devices of the RFA system.

Step S204: acquiring target ablation parameters of a target ablation task to which a task execution instruction points according to the recorded association relationship when the task execution instruction is triggered, and configuring the target ablation parameters into a RFA system.

Specifically, the task execution instruction may be sent to the configuration device from the server or other terminals. Alternatively, the task execution instruction may be triggered based on the user's operation on the task control interaction interface provided by the configuration device, or based on the user's pressing of a physical button set on the configuration device for triggering the task execution instruction.

The task execution instruction includes the identification information of the target ablation task. Optionally, the task execution instruction may further include identification information of the ablation object associated with the target ablation task.

According to the above identification information, corresponding target ablation parameters may be obtained by querying the above association relationship stored locally or on the database server, and then the target ablation parameters may be configured into the RFA system.

It may be understood that the target ablation parameters may be configuration parameters of at least one device of the RFA system, such as radio frequency power, ablation time, alarm value of the RFA control device, or liquid perfusion volume, perfusion time, alarm value of the injection pump, etc.

In an embodiment of the present application, by means of associating the ablation parameters extracted from the ablation scheme of the target file with the created ablation task by utilizing the target file to which the task creation instruction points, and configuring the target ablation parameters of the target ablation task to which the task execution instruction points into the radio frequency ablation system according to the recorded association relationship when the task execution instruction is triggered, automatic configuration of ablation parameters is realized. Since the configuration parameters are automatically extracted from the target file which needs to be generated only once by the configuration device and automatically configured into the radio frequency ablation system, on the basis of simplifying the parameter configuration operation, it can also avoid repetitive labor, save configuration time, and improve configuration efficiency.

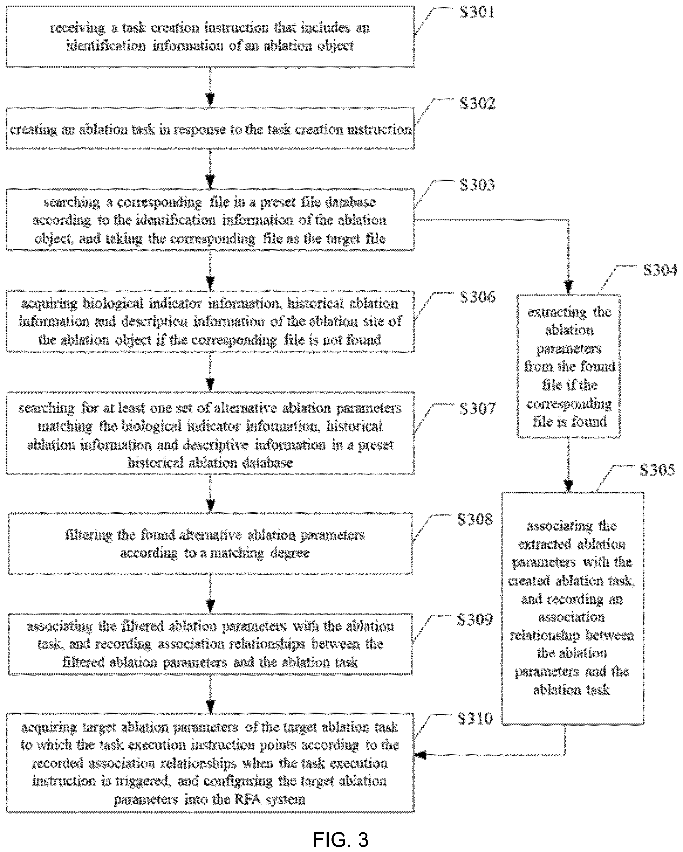

Referring to FIG. 3, which is a flowchart of an ablation parameter configuration method provided by another embodiment of the present application. The method may be implemented by a computer terminal, such as the parameter configuration device 70 or the RFA control device 10 or the injection pump 20 shown in FIG. 1, which is hereinafter referred to as a configuration device for ease of understanding. As shown in FIG. 3, the method includes:
Step S301, receiving a task creation instruction that includes an identification information of an ablation object;
Step S302, creating an ablation task in response to the task creation instruction;
Specifically, the task creation instruction may be sent to the configuration device from a server or other terminals. The other terminals may be, for example, personal computer devices used by doctors in various medical clinics. The identification information of the ablation object may be, for example, the name of the ablation object.

The configuration device responds to the task creation instruction, creates the ablation task, and assigns identification information to the created ablation task. This identification information is used to uniquely identify the created ablation task, which may be consisted of but not limited to at least one of numbers, letters, Chinese characters, and special symbols. Furthermore, the identification information may include, but is not limited to at least one of identification information of the ablation object, creating time and characteristics of an ablation site.

Optionally, in another embodiment, the server or other terminals may send the target file to the configuration device while sending the task creation instruction.
S303, searching a corresponding file in a preset file database according to the identification information of the ablation object, and taking the corresponding file as the target file;
S304, extracting the ablation parameters from the found file if the corresponding file is found;
S305, associating the extracted ablation parameters with the created ablation task, and recording an association relationship between the ablation parameters and the ablation task;
Specifically, the preset file database may be configured in the database server. The file database stores identification information of multiple ablation objects, multiple files, and corresponding relationships therebetween. The file includes the ablation scheme of the corresponding ablation object.

It may be understood that when an ablation object corresponds to multiple files, that is, when an ablation object has multiple ablation schemes, the file to which the most recently created ablation scheme belongs is taken as the found corresponding file.

In the file database, file corresponding to the identification information of the ablation object is searched. If a file corresponding to the identification information is found, the found file will be taken as the target file. Ablation parameters are extracted from the ablation scheme contained in the target file, and associated with the created ablation task. At the same time, the association relationship between the ablation parameters and the ablation task is recorded locally or on database server or on control device of the RFA system.

Optionally, a format of the target file may be but is not limited to any of word, txt, xml, CSV, DAT, DBF, MDB, ODB++files. Alternatively, the target file may be an image file of the user's handwritten prescription form. The configuration device may acquire the ablation scheme contained in the image file by utilizing file recognition technology.

It may be understood that association relationship of the configuration device related to the function of the configuration device may be recorded locally and other association relationships may be sent to corresponding devices for storage if the configuration device is a member of the RFA system. If the configuration device is not a member of the RFA system, the association relationship may be sent to corresponding devices of the RFA system for storage, respectively.

S306, acquiring biological indicator information, historical ablation information and description information of the ablation site of the ablation object if the corresponding file is not found;

Specifically, if the file corresponding to the identification information cannot be found in the file database according to the identification information of the ablation object, the biological indicator information, historical ablation information and description information of the ablation site of the ablation object may be acquired according to the identification information of the ablation object.

Optionally, the biological indicator information, historical ablation information and description information of the ablation site of the ablation object may be acquired from a preset personal information database of the ablation object according to the identification information of the ablation object.

Association relationship between the identification information of multiple ablation objects that have undergone or are about to undergo ablation operations and their corresponding biological indicator information, historical ablation information and the description information of the ablation site are stored on the personal information database of the ablation object, which may be, for example, configured in the database server.

Optionally, the biological indicator information of the ablation object may include but is not limited to at least one of the complications, weight, blood pressure within a preset time period, heart rate, blood sugar, blood lipids, vital capacity and oxygen saturation, age and gender of the ablation object.

Optionally, the historical ablation information of the ablation object may include but is not limited to the historical number of ablation operations performed on the ablation object. Furthermore, the historical ablation information may further include the ablation results corresponding to previous ablation operations performed on the ablation object, such as ablation site change information after the ablation operation.

Optionally, the description information of the ablation site of the ablation object is used to describe the characteristics of the ablation site, including but not limited to the position, size, shape and area of the ablation site.

S307, searching for at least one set of alternative ablation parameters matching the biological indicator information, historical ablation information and descriptive information in a preset historical ablation database;
The preset historical ablation database stores the identification information of multiple ablation objects as samples and association relationships with their corresponding biological indicator information, historical ablation information and description information, which may be for example, pre-configured in the database server.

Specifically, it is to search for alternative ablation parameters that match with the biological indicator information, historical ablation information and description information of the ablation site of the ablation object in the historical ablation database.

Among them, the closer the relevant information of the ablation object is to the corresponding information of the ablation object as a sample in the historical ablation database, the higher the matching degree. For example, the age of the ablation object is 30 years old. If the age of the ablation object as the sample in the historical ablation database is 30 years old, the matching degree is 100%. If the age of the ablation object as the sample is 10 years old, the matching degree is 30%.

S308, filtering the found alternative ablation parameters according to a matching degree;
Specifically, the found alternative ablation parameters may be filtered based on their matching degree. For example, the alternative ablation parameter with a matching degree less than a preset value may be removed from all of the found alternative ablation parameters.

Optionally, in another embodiment of the present application, filtering the found alternative ablation parameters according to the matching degree includes:
taking the ablation parameters in the alternative ablation parameters with the highest matching degree as the filtered ablation parameters; or taking the ablation parameters in the alternative ablation parameters with the matching degree greater than a preset first matching degree as the filtered ablation parameters.

Optionally, in another embodiment of the present application, filtering the found alternative ablation parameters according to the matching degree includes:
sorting the found alternative ablation parameters according to the number of matching items and the level of matching degree, the more items with the matching degree greater than a preset matching degree, the higher the ranking; and the higher the matching degree, the higher the ranking;
displaying the sorted alternative ablation parameters and outputting confirmation prompt information to prompt users to select;
taking the ablation parameters pointed by the user's selection operation as the filtered ablation parameters.

For example, if three sets of alternative ablation parameters A, B and C are found, wherein A set of alternative ablation parameters has a matching degree greater than 90% in terms of the aforementioned biological indicator information, historical ablation information and description information of ablation site, B set of alternative ablation parameters has a matching degree greater than 70% in terms of the aforementioned biological indicator information and historical ablation information, and C set of alternative ablation parameters has a matching degree greater than 85% in terms of the aforementioned biological indicator information, historical ablation information and description information of ablation site. So, it is sorted according to the number of matching items firstly, and then according to the matching degree, and the sorted alternative ablation parameters are A, C, and B from top to bottom. Then, the three sets of alternative ablation parameters are displayed on a display screen in the order of A, C, and B, and a confirmation prompt information is outputted, so that users can select from the three sets of alternative ablation parameters A, B and C according to the confirmation prompt information.

Like this, by sorting the alternative ablation parameters according to the number of matching items and the matching degree, the search results may have higher reference value, thereby helping users make faster choices and improving the accuracy of the selection results.

Furthermore, before filtering the found alternative ablation parameters according to the matching degree, a pre-filtering of the found alternative ablation parameters may be performed according to the ablation effects of other ablation objects, further improving the reference value of the search results. Specifically, this method further includes:
acquiring an ablation result information of the target ablation object corresponding to each set of alternative ablation parameters;
filtering out the ablation parameters that are less than a preset ablation result indicator from the found alternative ablation parameters according to the ablation result information; and executing the step of filtering the found alternative ablation parameters according to the matching degree based on the filtered alternative ablation parameters.

Specifically, the ablation result information may be obtained from the preset ablation result database according to the identification information of the ablation object, including but not limited to: the corresponding number of the ablation operations, the size, shape and area of the ablation site of the target ablation object after ablation operation, and the survival time of the target ablation object. Among them, the corresponding number of the ablation operations is used to indicate that this ablation result is the ablation result of which ablation task was performed on the target ablation object

The preset ablation result indicator may be found from the preset indicator database, including but not limited to: the corresponding number of ablation operations, the size, shape and area of the ablation site of the target ablation object after ablation operation, and the survival time of the target ablation object.

It may be understand that ablation parameters corresponding to ablation tasks with poor results may be filtered out from all of the found alternative ablation parameters based on the preset ablation result indicators, thereby further improving the reference value and pertinence of the search results.

S309, associating the filtered ablation parameters with the ablation task, and recording association relationships between the filtered ablation parameters and the ablation task;

Specifically, the method for recording the association relationships between the filtered ablation parameters and the ablation task may refer to the relevant description of step S305 above, which will not be repeated here.

S310, acquiring target ablation parameters of the target ablation task to which the task execution instruction points according to the recorded association relationships when the task execution instruction is triggered, and configuring the target ablation parameters into the RFA system.

Specifically, the task execution instruction may be sent to the configuration device from the server or other terminals. Alternatively, the task execution instruction may be triggered based on the user's operation on the task control interaction interface provided by the configuration device, or based on the user's pressing of a physical button set on the configuration device for triggering the task execution instruction.

The task execution instruction includes the identification information of the target ablation task. Optionally, the task execution instruction may further include identification information of the ablation object associated with the target ablation task.

According to the above identification information, corresponding target ablation parameters may be obtained by querying the above association relationships between the ablation parameters and the ablation task stored locally or on the database server, and then the target ablation parameters may be configured into the RFA system.

It may be understood that the target ablation parameters may be configuration parameters of at least one device of the RFA system, such as radio frequency power, ablation time, alarm value of the RFA control device, or liquid perfusion volume, perfusion time, alarm value of the injection pump, etc.

Optionally, when the above association relationships are stored on the configuration device locally, the configuration device may configure the target ablation parameters into the RFA system by sending the parameter configuration instruction and the target ablation parameters stored locally to a corresponding device.

Optionally, when the above association relationships are stored on the database server, the configuration device may configure the target ablation parameters into the RFA system by sending the parameter configuration instruction, identification information of the target ablation task, and a storage location of the above association relationships to a corresponding device. The corresponding device obtains the corresponding target ablation parameters from the database server based on the received information and configures them locally.

Optionally, in order to ensure the accuracy of target ablation parameter configuration, in another embodiment of this application, the method further includes steps before configuring the target ablation parameters into the RFA system:

displaying the target ablation parameters through a preset display interface, and marking the target ablation parameters obtained through matching on the display interface according to a preset mark method;

modifying the target ablation parameters according to the user's modification operation on the display interface, and executing the step of configuring the target ablation parameters into the RFA system based on the modified target ablation parameters.

Specifically, the preset mark method may include but is not limited to adding text, graphics, or animation annotations, or displaying the target ablation parameters obtained through matching in specific colors or fonts.

It may be understood that the target ablation parameters obtained through the above matching operation are determined according to partial individual data of the ablation object to which the ablation task is to be executed and historical ablation data of other ablation objects, and the actual situation of the ablation object is constantly changing. Therefore, by means of displaying the target ablation parameters before configuration, and determining the final ablation parameters to be configured according to the user's operation to the displayed target ablation parameters, the accuracy of ablation parameters may be further improved and configuration errors may be further avoided. At the same time, by means of marking the matched target ablation parameters in the display interface, the effect of reminding users to pay attention may be achieved.

Furthermore, since the modification of ablation parameters requires the modifier to have a certain degree of professionalism, in order to avoid the target ablation parameters being mistakenly modified or tampered with, it further includes steps before modifying the target ablation parameters according to the user's modification operation:
acquiring user's identification information;
determining whether the user has been pre-authorized according to the acquired identification information and a preset authorization information;
executing the above step of modifying the target ablation parameters according to the user's modification operation when the user is pre-authorized.

Optionally, the form of the user's identification information and authorization information may include, but is not limited to, any one or any combinations of digital passwords, graphic passwords and the user's biometric information (such as voiceprint information, fingerprint information, facial information, iris information).

Optionally, in another embodiment of this application, the configuration device may obtain partial ablation parameters from the target file through steps S303 to S305, and then obtain the remaining ablation parameters by utilizing the historical ablation database through steps S306 to S309.

Specifically, it further includes steps after step S305: obtaining the biological indicator information, historical ablation information, and description information of the ablation site of the ablation object when the extracted ablation parameters cannot be used to complete the parameter configuration of all devices to be configured in the RFA system, and executing steps S307 and S309 based on the target device in the RFA system until the ablation parameters corresponding to the target device are filtered and associated with the created ablation task, wherein the target device cannot use the extracted ablation parameters for parameter configuration. That is to say, some of the associated ablation parameters are derived from the target file and some are based on historical ablation databases. In this way, the reference value of the ablation parameters may be further improved, and in scenarios where the final determination of parameter configuration is required by the user, it may help the user complete modification of the configuration parameters more quickly.

It may be understood that the above association relationships actually include association relationships between the ablation parameters, devices and ablation task corresponding to the ablation parameters, and ablation object corresponding to the ablation task.

Taking FIG. 1 as an example, assume that the ablation parameters to be associated include parameters of the RFA catheter 60 and the injection pump 20. Firstly, it is to search for a corresponding target file through steps S303 to S305. If only the relevant parameters of the RFA catheter 60 are extracted according to the found target file, it is to obtain the relevant parameters of the injection pump 20 through steps S306 to S309.

In the embodiments of this application, on one aspect, the ablation parameters extracted from the ablation scheme of the target file are associated with the created ablation task by means of utilizing the target file to which the task creation instruction points, and the target ablation parameters of the target ablation task to which the task execution instruction points are configured into the radio frequency ablation system according to the recorded association relationship when the task execution instruction is triggered, thereby realizing automatic configuration of ablation parameters. Since the configuration parameters are automatically extracted from the target file which needs to be generated only once by the configuration device and configured into the radio frequency ablation system, on the basis of simplifying the parameter configuration operation, it can also avoid repetitive labor, save configuration time, and improve configuration efficiency. On one aspect, suitable ablation parameters are automatically matched from the historical ablation data of other ablation objects according to the biological indicator information, historical ablation information, and description information of the ablation site of the ablation object associated with the created ablation task when there is no target file, which realizes the automatic configuration of ablation parameters based on historical ablation big data, and thus improves the convenience of ablation parameter configuration.

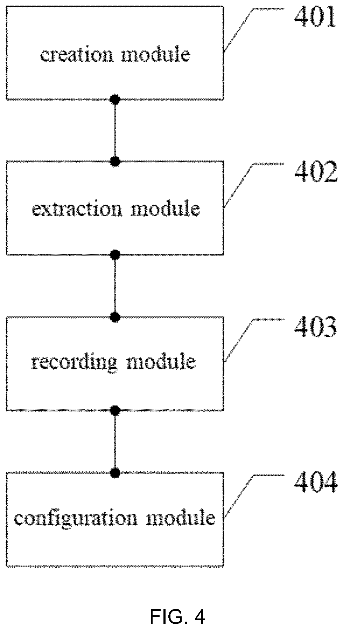

Referring to FIG. 4, which is a schematic view of an ablation parameter configuration device provided by an embodiment of the present application. For ease of description, only the parts related to the embodiments of the present application are shown. This device may be installed in the injection pump 20 shown in FIG. 1, or in the RFA control device 10, or in other computer devices. This device includes a creation module 401, an extraction module 402, a recording module 403 and a configuration module 404.

The creation module 401 is configured for creating an ablation task in response to a task creation instruction;

the extraction module 402 is configured for acquiring a target file to which the task creation instruction points and extracting ablation parameters from the target file, wherein the target file includes an ablation scheme of an ablation object to which the task creation instruction points;

the recording module 403 is configured for associating the extracted ablation parameters with the ablation task and recording the association relationship between the ablation parameters and the ablation task; and

the configuration module 404 is configured for acquiring target ablation parameters of a target ablation task to which a task execution instruction points according to the recorded association relationship when the task execution instruction is triggered and configuring the target ablation parameters into a radio frequency ablation system.

Optionally, this device further includes:
a receiving module configured for receiving the task creation instruction which includes identification information of the ablation object.

The extraction module 402 is further configured to search for corresponding file in the file database based on the identification information to serve as the target file and extract the ablation parameter from the found corresponding file.

Optionally, this device further includes:
an acquisition module, configured for acquiring biological indicator information, historical ablation information, and description information of an ablation site of the ablation object when the corresponding file cannot be found in the file database;
a search module, configured for searching for at least one set of alternative ablation parameters matching the biological indicator information, historical ablation information, and description information in the historical ablation database;
a filtering module, configured for filtering the found alternative ablation parameters based on the matching degree.

The recording module 403 is further used to associate the filtered ablation parameters with the ablation task, and record the association relationships between the filtered ablation parameters and the ablation task.

Optionally, the filtering module is further used to select the ablation parameters with the highest matching degree or a matching degree greater than the preset first matching degree from the alternative ablation parameters as the selected ablation parameter.

Optionally, the filtering module is further used to sort the alternative ablation parameters according to the number of matching items and the level of matching degree, wherein more items with a matching degree greater than the preset matching degree, the ranking is higher; and the higher the matching degree, the ranking is higher. The sorted alternative ablation parameters are displayed and confirmation prompt information is outputted to prompt users to make a selection. The ablation parameters pointed by the user's selection are taken as the filtered ablation parameters.

Optionally, this device further includes:
an ablation result acquisition module, which is used to obtain the ablation result information of the target ablation object corresponding to each set of alternative ablation parameters.

The filtering module is further used to filter out the alternative ablation parameters that are less than the preset ablation result indicators from the found alternative ablation parameters based on the ablation result information. Based on the filtered alternative ablation parameters, the step of filtering the found alternative ablation parameters based on matching degree is executed.

Optionally, the biological indicator information includes at least one of the complications, weight, blood pressure within a preset time period, heart rate, blood sugar, blood lipids, vital capacity and oxygen saturation, age and gender of the ablation object.

The historical ablation information includes the historical number of ablation operations performed on the ablation object.

The description information includes the position, size, shape and area of the ablation site.

The ablation result information includes the corresponding number of the ablation operations, the size, shape and area of the ablation site of the target ablation object after ablation operation, and the survival time of the target ablation object.

The preset ablation result indicators include the corresponding number of the ablation operations, the reference range of the size, shape and area of the ablation site after ablation operation, and the reference survival time.

Optionally, this device further includes:
a display module, which is used to display the target ablation parameters and mark the matched target ablation parameters in a display interface according to the preset mark method.

The configuration module 404 is further used to modify the target ablation parameters based on the user's modification operation on the display interface, and execute the step of configuring the target ablation parameters into the RFA system based on the modified target ablation parameters.

Optionally, this device further includes:
an identification information acquisition module configured for obtaining the identification information of the user, and
a judgment module for determining whether the user has been pre-authorized based on the obtained identification information and preset authorization information.

The configuration module 404 is also used to execute the step of modifying the target ablation parameters according to the user's modification operation if the user is pre-authorized.

Optionally, this acquisition module is also used to obtain the biological indicator information, historical ablation information, and description information of the ablation site of the ablation object when the extracted ablation parameters cannot be used to complete the parameter configuration of all devices to be configured in the RFA system.

The specific process of implementing the respective functions of each module described above may refer to the relevant content in the embodiments as shown in FIG. 2 and FIG. 3, which will not be repeated here.

In the embodiments of this application, on one aspect, the ablation parameters extracted from the ablation scheme of the target file are associated with the created ablation task by means of utilizing the target file to which the task creation instruction points, and the target ablation parameters of the target ablation task to which the task execution instruction points are configured into the radio frequency ablation system according to the recorded association relationship when the task execution instruction is triggered, thereby realizing automatic configuration of ablation parameters. Since the configuration parameters are automatically extracted from the target file which needs to be generated only once by the configuration device and configured into the radio frequency ablation system, on the basis of simplifying the parameter configuration operation, it can also avoid repetitive labor, save configuration time, and improve configuration efficiency. On one aspect, suitable ablation parameters are automatically matched from the historical ablation data of other ablation objects according to the biological indicator information, historical ablation information, and description information of the ablation site of the ablation object associated with the created ablation task when there is no target file, which realizes the automatic configuration of ablation parameters based on historical ablation big data, and thus improves the convenience of ablation parameter configuration.

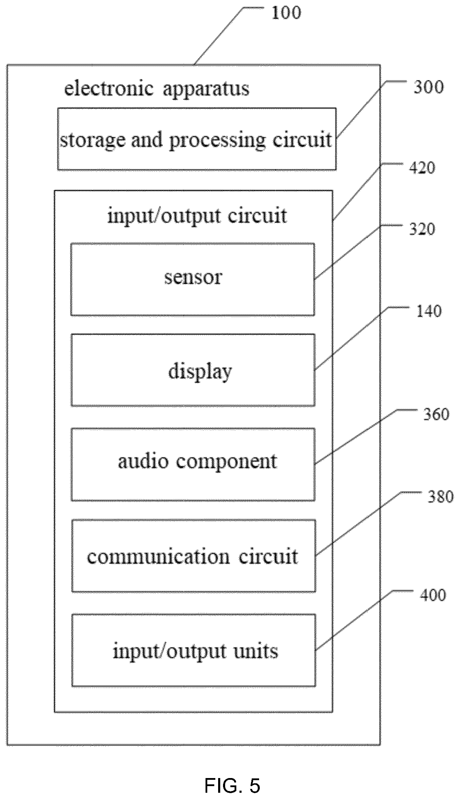

Referring to FIG. 5, which is a schematic view of a hardware structure of an electronic apparatus provided by an embodiment of the present application.

Exemplarily, the electronic apparatus may be any one of various types of computer system devices that are non-movable or movable or portable and perform wireless or wired communication. Particularly, the electronic apparatus may be a desktop computer, a server, a mobile phone or a smart phone (for example, an iPhone-TM-based phone, an Android-TM-based phone), a portable game device (for example, Nintendo DS TM, PlayStation Portable TM, Gameboy Advance TM, iPhone TM), a laptop computer, a PDA, a portable Internet device, a music player and a data storage device, and other handheld devices such as watches, earphones, pendants, and earphones. The electronic apparatus may further be other wearable devices (for example, electronic glasses, electronic clothes, electronic bracelets, electronic necklaces, electronic tattoos, electronic apparatuses or head-mounted devices (HMD) of the smart watches).

The electronic apparatus may be any one of multiple electronic apparatuses, including but not limited to a cellular phone, a smart phone, other wireless communication devices, a personal digital assistant, an audio player, other media players, a music recorder, a video recorder, a camera, other media recorders, a radio, a medical device, a vehicle transportation instrument, a calculator, a programmable remote control, a pager, a laptop computer, a desktop computer, a printer, a netbook computer, a personal digital assistant (PDA), a portable multimedia player (PMP), a motion picture experts group (MPEG-1 or MPEG-2) audio layer 3 (MP3) player, a portable medical device and a digital camera, and a combination thereof.

In some cases, the electronic apparatus may perform multiple functions (for example, playing music, displaying videos, storing pictures, and receiving and transmitting phone calls). If desired, the electronic apparatus may be a portable device such as a cell phone, a media player, other handheld devices, a wrist watch device, a pendant device, an earpiece device, or other compact portable devices.

As shown in FIG. 5, the electronic apparatus 100 may include a control circuit, wherein the control circuit may include a storage and processing circuit 300. The storage and processing circuit 300 may include a memory, such as a hard disk drive memory, a non-volatile memory (such as a flash memory or other electronically programmable restricted deletion memory configured to form a solid-state drive), and a volatile memory (for example, a static or dynamic random access memory and the like), and the like, which are not limited in the embodiment of the present application. The processing circuit in the storage and processing circuit 300 may be configured to control the operation of the electronic apparatus 100. The processing circuit may be implemented based on one or more microprocessors, microcontrollers, digital signal processors, baseband processors, power management units, audio codec chips, application specific integrated circuits, display driver integrated circuits, and the like.

The storage and processing circuit 300 may be configured to execute software in the electronic apparatus 100, such as an Internet browsing application, a Voice over Internet Protocol (VOIP) telephone calling application, an email application, a media playback application, an operating system function, and the like. The software may be configured to perform some control operations, for example, image capture based on a camera, ambient light measurement based on an ambient light sensor, proximity sensor measurement based on a proximity sensor, and information display function realized based on a status indicator such as a status indicator lamp of a LED, touch event detection based on a touch sensor, a function associated with displaying information on multiple (for example, layered) displays, an operation associated with performing a wireless communication function, an operation associated with acquiring and generating an audio signal, a control operation associated with acquiring and processing of button press event data, and other functions in the electronic apparatus 100, which are not limited in the embodiment of the present application.

Further, the memory stores an executable program code, and a processor coupled with the memory calls the executable program code stored in the memory to perform the above ablation parameter configuration method for syringe pump described in the embodiments as shown in FIG. 2 and FIG. 3.

Among them, the executable program code includes various modules in the ablation parameter configuration apparatus described in the embodiment shown in FIG. 4, for example, the creation module 401, the extraction module 402, the recording module 403 and the configuration module 404.

The creation module 401 is configured for creating an ablation task in response to a task creation instruction;
the extraction module 402 is configured for acquiring a target file to which the task creation instruction points and extracting ablation parameters from the target file, wherein the target file includes an ablation scheme of an ablation object to which the task creation instruction points;
the recording module 403 is configured for associating the extracted ablation parameters with the ablation task and recording the association relationship between the ablation parameters and the ablation task; and
the configuration module 404 is configured for acquiring target ablation parameters of a target ablation task to which a task execution instruction points according to the recorded association relationship when the task execution instruction is triggered and configuring the target ablation parameters into a radio frequency ablation system.

The electronic apparatus 100 may further include an input/output circuit 420. The input/output circuit 420 may be configured to enable the electronic apparatus 100 to input and output data, that is, to allow the electronic apparatus 100 to receive data from an external device and further allow the electronic apparatus 100 to output data from the electronic apparatus 100 to the external device. The input/output circuit 420 may further include a sensor 320. The sensor 320 may include an ambient light sensor, a proximity sensor based on light and capacitive, and a touch sensor (for example, a light-based touch sensor and/or a capacitive touch sensor, wherein the touch sensor may be a part of a touch display screen, or may be independently used as a touch sensor), an acceleration sensor, other sensors and the like.

The input/output circuit 420 may further include one or more displays, such as a display 140. The display 140 may include one or a combination of a liquid crystal display, an organic light emitting diode display, an electronic ink display, a plasma display, and a display using other display technologies. The display 140 may include a touch sensor array (i.e., the display 140 may be a touch display screen). The touch sensor can be a capacitive touch sensor formed by an array of transparent touch sensor electrodes (for example, indium tin oxide (ITO) electrodes), or a touch sensor formed using other touch technologies, such as sonic touch, pressure-sensitive touch, resistance touch, optical touch, and the like, which are not limited in the embodiment of the present application.

The electronic apparatus 100 may further include an audio component 360. The audio component 360 may be configured to provide audio input and output functions for the electronic apparatus 100. The audio component 360 in the electronic apparatus 100 may include a speaker, a microphone, a buzzer, a tone generator, and other components for generating and detecting sounds.

The communication circuit 380 may be configured to provide the electronic apparatus 100 with the ability to communicate with an external device. The communication circuit 380 may include an analog and digital input/output interface circuit, and a wireless communication circuit based on a radio frequency signal and/or an optical signal. The wireless communication circuit in the communication circuit 380 may include a radio frequency transceiver circuit, a power amplifier circuit, a low noise amplifier, a switch, a filter and an antenna. For example, the wireless communication circuit in the communication circuit 380 may include a circuit for supporting near field communication (NFC) by transmitting and receiving a near-field coupled electromagnetic signal. For example, the communication circuit 380 may include a near-field communication antenna and a near-field communication transceiver. The communication circuit 380 may further include a cellular phone transceiver and an antenna, a wireless local area network transceiver circuit and an antenna, and the like.

The electronic apparatus 100 may further include a battery, a power management circuit and other input/output units 400. The input/output unit 400 may include a button, a joystick, a click wheel, a scroll wheel, a touch pad, a keypad, a keyboard, a camera, a light emitting diode, and other status indicators.

The user may input a command through the input/output circuit 420 to control the operation of the electronic apparatus 100, and may use the output data of the input/output circuit 420 to realize the reception of status information and other outputs from the electronic apparatus 100.

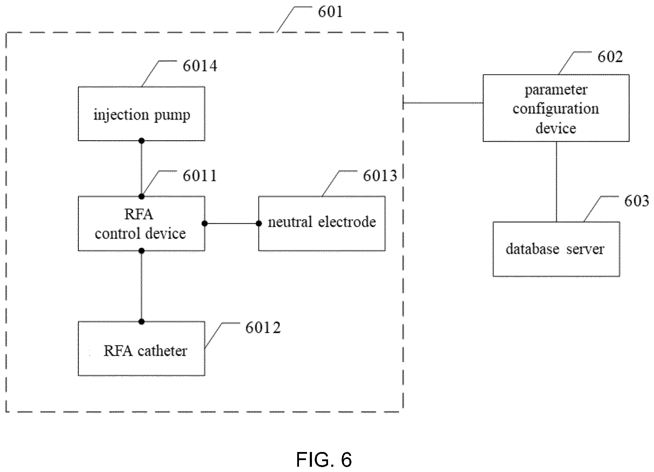

Further, as shown in FIG. 6, an embodiment of the present application further provides an ablation parameter configuration system. For ease of description, only the parts related to the embodiments of the present application are shown. The ablation parameter configuration system includes RFA system 601, a parameter configuration device 602 and a database server 603.

The RFA system 601 includes a RFA control device 6011, a RFA catheter 6012, a neutral electrode 6013 and an injection pump 6014; and the database server 603 may be a single server or a distributed server cluster consisted of a plurality of servers.

The parameter configuration device 602 is configured for executing steps of the ablation parameter configuration method provided in the embodiments as shown in FIG. 2 and FIG. 3.

The database server 603 is used to store various databases and provide data query services based on these databases for devices of the parameter configuration device 602 and the RFA system 601. The various databases may include, but are not limited to, the file database, historical ablation database, ablation object personal information database, ablation result database and indicator database in the embodiments as shown in FIG. 2 and FIG. 3.

The specific process of implementing the respective functions of the RFA system 601, the parameter configuration device 602 and the database server 603 may refer to the relevant content in the embodiments as shown in FIG. 1 to FIG. 5, which will not be repeated here.

In an embodiment of the present application, by means of associating the ablation parameters extracted from the ablation scheme of the target file with the created ablation task by utilizing the target file to which the task creation instruction points, and configuring the target ablation parameters of the target ablation task to which the task execution instruction points into the radio frequency ablation system according to the recorded association relationships when the task execution instruction is triggered, automatic configuration of ablation parameters is realized. Since the configuration parameters are automatically extracted from the target file which needs to be generated only once by the configuration device and automatically configured into the radio frequency ablation system, on the basis of simplifying the parameter configuration operation, it can also avoid repetitive labor, save configuration time, and improve configuration efficiency.

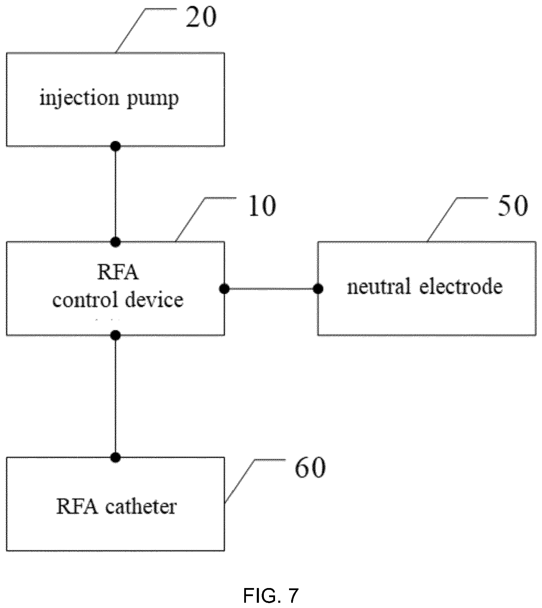

Further, as shown in FIG. 7, an embodiment of the present application further provide a RFA system that includes a RFA control device 10, an injection pump 20, a neutral electrode 50 and a RFA catheter 60.

The RFA catheter 60 and the neutral electrode 50 are electrically connected to the RFA control device 10, and the RFA control device 10 is electrically coupled to the injection pump 20.

The RFA control device 10 or the injection pump 20 is configured for executing steps of the ablation parameter configuration method provided in the embodiments as shown in FIG. 2 and FIG. 3.

The RFA catheter 60 is configured for executing ablation operations on the ablation object according to the control instructions of the RFA control device 10 and according to corresponding target ablation parameters.

The injection pump 20 configured for executing perfusion operations on the ablation object according to the corresponding target ablation parameters.

The specific process of implementing the respective functions of the RFA control device 10, the injection pump 20, the neutral electrode 50 and the RFA catheter 60 may refer to the relevant content in the embodiments as shown in FIG. 1 to FIG. 5, which will not be repeated here.

In an embodiment of the present application, by means of associating the ablation parameters extracted from the ablation scheme of the target file with the created ablation task by utilizing the target file to which the task creation instruction points, and configuring the target ablation parameters of the target ablation task to which the task execution instruction points into the radio frequency ablation system according to the recorded association relationships when the task execution instruction is triggered, automatic configuration of ablation parameters is realized. Since the configuration parameters are automatically extracted from the target file which needs to be generated only once by the configuration device and automatically configured into the radio frequency ablation system, on the basis of simplifying the parameter configuration operation, it can also avoid repetitive labor, save configuration time, and improve configuration efficiency.

Further, embodiments of the present application further provide a computer-readable storage medium. The computer-readable storage medium may be provided in the electronic apparatus of each of the foregoing embodiments, and the computer-readable storage medium may be a memory in the storage and processing circuit 300 of the embodiment as shown in FIG. 5. A computer program is stored on the computer-readable storage medium, and is executed by the processor to realize the ablation parameter configuration method described in the foregoing embodiments as shown in FIG. 2 and FIG. 3. Further, the computer-storable storage medium may further be a U disk, a mobile hard disk, a read-only memory (ROM), a RAM, a magnetic disk or an optical disk, and other various media that may store the program code.

In the several embodiments provided in the present application, it should be understood that the disclosed apparatus and method may be implemented in other ways. For example, the embodiments of the apparatus described above are merely illustrative. For example, the division of the modules is only a logical function division, or other divisions in practical implementations, for example, multiple modules or components may be combined or may be integrated into another system, or some features may be ignored or not performed. In addition, the displayed or discussed mutual coupling or direct coupling or communication connection may be indirect coupling or communication connection through some interfaces, apparatuses or modules, and may be in electrical, mechanical or other forms.

The modules described as separate components may or may not be physically separated, and the components displayed as modules may or may not be physical modules, that is, they may be located in one place, or they may be distributed onto multiple network modules. Some or all of the modules may be selected according to actual needs to achieve the objectives of the solutions of the embodiments.

In addition, the functional modules in the various embodiments of the present application may be integrated into one processing module, or each module may exist alone physically, or two or more modules may be integrated into one module. The above-mentioned integrated modules may be implemented in the form of hardware or a software functional module.

If the integrated module is implemented in the form of the software function module and sold or used as an independent product, it may be stored in a computer-readable storage medium. Based on such understanding, the technical solution of the present application substantively, or a part thereof making a contribution to the prior art, or all or part of the technical solution may be embodied in the form of a software product stored in a readable storage medium, and the readable storage medium includes several instructions to enable a computer device (which may be a personal computer, a server, or a network device) to execute all or part of the step of the methods described in the various embodiments of the present application. The above-mentioned readable storage medium includes a U disk, a mobile hard disk, a ROM, a RAM, a magnetic disk or an optical disk, and other media that may store the program code.

It should be noted that for the foregoing method embodiments, for simplicity of description, they are all expressed as a series of action combinations, but those skilled in the art should appreciate that the present application is not limited by the described sequence of actions. Because according to the present application, some steps may be performed in other sequences or simultaneously, and secondly, those skilled in the art should further appreciate that the embodiments described in the specification are all preferred embodiments, and the involved actions and modules are not necessarily all required by the present application.

In the above-mentioned embodiments, descriptions of the embodiment have particular emphasis respectively. For parts that are not described in detail in a certain embodiment, reference may be made to related descriptions of other embodiments.

The above is a description of the ablation parameter configuration method, apparatus and system, and the computer-readable storage medium according to the present application. For those skilled in the art, according to the ideas of the embodiments of the present application, changes may be made to specific implementations and application scopes. In summary, the content of this specification should not be construed as a limitation on the present application.

## Claims

1. An ablation parameter configuration method, applied to a computer terminal, comprising:
creating an ablation task in response to a task creation instruction;
acquiring a target file to which the task creation instruction points and extracting ablation parameters from the target file, wherein the target file comprises an ablation scheme of an ablation object to which the task creation instruction points;
associating the extracted ablation parameters with the ablation task, and recording an association relationship between the ablation parameters and the ablation task;
acquiring target ablation parameters of a target ablation task to which a task execution instruction points according to the recorded association relationship when the task execution instruction is triggered, and configuring the target ablation parameters into a radio frequency ablation system.

2. The method according to claim 1, wherein before the step of creating the ablation task in response to the task creation instruction, the method comprises:
receiving the task creation instruction that comprises an identification information of the ablation object;
the step of acquiring the target file to which the task creation instruction points and extracting ablation parameters from the target file comprising:
searching a corresponding file in a preset file database according to the identification information and taking the corresponding file as the target file.

3. The method according to claim 2, wherein the method further comprises:
acquiring biological indicator information, historical ablation information and description information of an ablation site of the ablation object when the corresponding file is not found in the file database;
searching for at least one set of alternative ablation parameters matching the biological indicator information, historical ablation information and descriptive information in a preset historical ablation database;
filtering the found alternative ablation parameters according to a matching degree;
associating the filtered ablation parameters with the ablation task, and recording association relationships between the filtered ablation parameters and the ablation task.

4. The method according to claim 3, wherein the step of filtering the found alternative ablation parameters according to matching degree comprises:
taking the ablation parameters in the alternative ablation parameters with the highest matching degree as the filtered ablation parameters; or taking the ablation parameters in the alternative ablation parameters with the matching degree greater than a preset first matching degree as the filtered ablation parameters.

5. The method according to claim 3, wherein the step of filtering the found alternative ablation parameters according to the matching degree comprises:
sorting the found alternative ablation parameters according to the number of matching items and the level of matching degree, the more items with the matching degree greater than a preset matching degree, the higher the ranking; and the higher the matching degree, the higher the ranking;
displaying the sorted alternative ablation parameters and outputting confirmation prompt information to prompt the user to select;
taking the ablation parameters pointed by the user's selection operation as the filtered ablation parameters.

6. The method according to any one of claims 3-5, wherein
the biological indicator information comprises at least one of the complications, weight, blood pressure within a preset time period, heart rate, blood sugar, blood lipids, vital capacity and oxygen saturation, age and gender of the ablation object;
the historical ablation information comprises the historical number of ablation operations performed on the ablation object;
the description information comprises position, size, shape and area of the ablation site.

7. The method according to any one of claims 3-5, wherein before the step of filtering the found alternative ablation parameters according to the matching degree, the method further comprises:
acquiring an ablation result information of the target ablation object corresponding to each set of alternative ablation parameters;
filtering out the ablation parameters that are less than a preset ablation result indicator from the found alternative ablation parameters according to the ablation result information; and executing the step of filtering the found alternative ablation parameters according to the matching degree based on the filtered alternative ablation parameters.

8. The method according to claim 7, wherein
the biological indicator information comprises at least one of the complications, weight, blood pressure within a preset time period, heart rate, blood sugar, blood lipids, vital capacity and oxygen saturation, age and gender of the ablation object;
the historical ablation information comprises the historical number of ablation operations performed on the ablation object;
the description information comprises position, size, shape and area of the ablation site;
the ablation result information comprises corresponding number of the ablation operations, size, shape and area of the ablation site of the target ablation object after ablation operation, and survival time of the target ablation object;
the preset ablation result indicator comprises the corresponding number of the ablation operations, reference range of the size, shape and area of the ablation site after ablation operation, and reference survival time.

9. The method according to any one of claims 3-5, wherein before the step of configuring the target ablation parameters into the radio frequency ablation system, the method further comprises:
displaying the target ablation parameters, and marking the target ablation parameters obtained through matching on a display interface according to a preset marking method;
modifying the target ablation parameters according to the user's modification operation on the display interface, and executing the step of configuring the target ablation parameters into the radio frequency ablation system based on the modified target ablation parameters.

10. The method according to claim 9, wherein before the step of modifying the target ablation parameters according to the user's modification operation on the display interface, the method further comprises:
acquiring user's identification information;
determining whether the user has been pre-authorized according to the acquired identification information and a preset authorization information;
executing the step of modifying the target ablation parameters according to the user's modification operation when the user is pre-authorized.

11. The method according to claim 9, wherein after the step of associating the extracted ablation parameters with the ablation task, and recording the association relationship between the ablation parameters and the ablation task, the method further comprises:
acquiring the biological indicator information, historical ablation information, and description information of the ablation site of the ablation object when the extracted ablation parameters are not capable of completing parameter configuration of all devices to be configured of the radio frequency ablation system,
executing the step of searching for at least one set of alternative ablation parameters matching the biological indicator information, historical ablation information and descriptive information in the preset historical ablation database based on the target device of the radio frequency ablation system until the ablation parameters corresponding to the target device are filtered, wherein the target device is not capable of performing parameter configuration by utilizing the extracted ablation parameters.

12. An ablation parameter configuration device comprising:
a creation module, configured for creating an ablation task in response to a task creation instruction;
an extraction module, configured for acquiring a target file to which the task creation instruction points and extracting ablation parameters from the target file, wherein the target file comprises an ablation scheme of an ablation object to which the task creation instruction points;
a recording module, configured for associating the extracted ablation parameters with the ablation task and recording an association relationship between the ablation parameters and the ablation task; and
a configuration module, configured for acquiring target ablation parameters of a target ablation task to which a task execution instruction points according to the recorded association relationship when the task execution instruction is triggered and configuring the target ablation parameters into a radio frequency ablation system.

13. An electronic apparatus comprising a memory and a processor;
the memory storing executable program codes thereon;
the processor being electrically coupled to the memory, calling the executable program codes stored on the memory, and executing the ablation parameter configuration method defined in any one of claims 1-11.

14. An ablation parameter configuration system comprising a radio frequency ablation system, a parameter configuration device and a database server;
the radio frequency ablation system comprising a radio frequency ablation control device, a radio frequency ablation catheter, a neutral electrode and an injection pump;
the parameter configuration device being configured for executing steps of the ablation parameter configuration method defined in any one of claims 1-11.

15. A radio frequency ablation system comprising a radio frequency ablation control device, a radio frequency ablation catheter, a neutral electrode and an injection pump;
the radio frequency ablation control device or the injection pump being configured for executing steps of the ablation parameter configuration method defined in any one of claims 1-11.

16. A computer-readable storage medium on which a computer program is stored, **characterized in that** the computer program, when executed by a processor, implements the ablation parameter configuration method defined in any one of claims 1-11.
